# EUROPÄISCHE PATENTANMELDUNG

(11) **EP 4 744 587 A1**
(43) Veröffentlichungstag der Anmeldung: **20.05.2026**
(21) Anmeldenummer: 25212874.9
(22) Anmeldetag: 01.11.2025
(51) Int. Cl.: A61B 5/107, A61B 5/1172, A61B 5/00, A61F 5/058, A61F 5/10, G06V 40/10

(54) **VORRICHTUNG ZUR FOTOGRAFISCHEN ERFASSUNG DER PAPILLARLEISTEN EINER FINGERKUPPE**

(30) Priorität: 04.11.2024 CH 11912024
(71) Anmelder: Mujic, Senad, 4059 Basel (CH)
(72) Erfinder: Mujic, Senad, 4059 Basel (CH)
(74) Vertreter: Herrmann, Johanna

(57) **Zusammenfassung**

Eine Vorrichtung (10) zur fotografischen Erfassung der Papillarleisten einer Fingerkuppe einer verstorbenen Person umfasst einen Messkopf (5), wobei der Messkopf (5) zur Aufnahme eines Fingerendglieds ausgebildet ist. Der Messkopf (5) enthält einen Mantel (4), wobei der Mantel (4) einen ersten Endbereich (1) und einen zweiten Endbereich (2) und eine Mittenachse (3) enthält. Der erste Endbereich (1) des Messkopfs (5) ist als eine zumindest teilweise geschlossene Oberfläche ausgebildet. Der zweite Endbereich (2) des Messkopfs (5) umfasst einen Messring (6). Ein Messfenster (7) ist im Mantel (4) des Messkopfs (5) zwischen dem ersten Endbereich (1) und dem zweiten Endbereich (2) angeordnet. Ein Griffelement (8) erstreckt sich vom zweiten Endbereich (2) des Messkopfs (5) im Wesentlichen in Richtung der Mittenachse (3).

## Beschreibung

### Hintergrund

Die vorliegende Erfindung betrifft eine Vorrichtung zur fotografischen Erfassung der Papillarleisten einer Fingerkuppe einer verstorbenen Person, nachfolgend auch als Leichendaktyloskopie-Kelle bezeichnet.

Wenn eine Person verstirbt, setzt bereits nach 2-6 Stunden die Leichenstarre ein. Aufgrund des Auftretens biochemischer Prozesse im Körper wie die Einstellung der Produktion von Adenosintriphosphat, welches zur Muskelentspannung notwendig ist, führt dies unweigerlich zur Versteifung der Muskelgruppen. Die Hände der verstorbenen Person ballen sich dabei zu Fäusten, wobei die notwendigen Papillarleisten, welche sich an den Innenseiten der Fingerendglieder befinden, durch die Finger selbst in der geballten Faust verdeckt werden. Diese Leichenstarre kann mehrere Tage andauern. Die schnellste polizeiliche Identifizierung führt über die Papillarleisten der Finger, vorwiegend Zeigefinger. Andere polizeiliche Prozesse bedürfen aufwändigerer Recherche sowie Konsultationen von ärztlichen Unterlagen und medizinischen Untersuchungen der Leiche beispielsweise durch das Institut für Rechtsmedizin (IRM).

### Stand der Technik

Aus der DE 370708 A ist eine Vorrichtung zur Videoerfassung des Papillarleistenmusters an der Fingerkuppe eines Menschen mit einem Video-Bildaufnehmer gezeigt. Der Video-Bildaufnehmer hat eine lichtempfindliche Sensorfläche. Eine faseroptische Linse mit einer Auflage für den Finger ermöglicht die Abbildung des Papillarleistenmusters auf der Sensorfläche. Mit dieser Vorrichtung kann ein Fingerabdruck einer lebenden Person abgebildet werden, welche in der Lage ist, den Finger in der Vorrichtung zu platzieren.

Im Dokument DE 37 12 617 ist eine Vorrichtung zur Erstellung von Fingerabdrücken gezeigt, mittels welcher ein in der Vorrichtung eingespannter Finger mit einer Farbwalze eingeschwärzt wird und anschliessend mittels einer Transportwalze ein Transportband am eingeschwärzten Finger abgewälzt wird, um den Fingerabdruck auf das Transportband zu übertragen. Das Transportband kann eine Vorschubbewegung ausführen. Mit dieser Vorrichtung können Fingerabdrücke von allen Fingern einer Hand sequenziell abgenommen werden. Mit dieser Vorrichtung wird ein Fingerabdruck in Papierform erstellt. Der Finger wird hierzu in einer entsprechenden Öffnung der Vorrichtung arretiert, sodass der Finger während des Farbauftrags oder während des Abnehmens auf das Transportband nicht verrutschen kann. Ein Finger einer verstorbenen Person, bei welcher die Leichenstarre eingetreten ist, kann mittels eines herunterklappbaren Griffs gestreckt werden. Diese Vorrichtung eignet sich allerdings nicht, um den Finger zu fotografieren, da sich der Finger im Inneren der Vorrichtung zwischen der Transportwalze, dem Transportband und der Farbwalze befindet, die um den Finger herumgeführt werden, sodass zumindest Teile des Fingers verdeckt sind.

Die Papillarleisten müssen für die heutigen Anforderungen sauber und ohne grossen Verzug mit einem Massstab fotografisch dokumentiert werden, damit bei der Vergleichsarbeit die genaue Grösse und Abstände der Merkmale nachvollzogen werden kann. Die Aufgabe der Identifikation obliegt in erster Linie und überwiegend der Kriminaltechnik/Forensik. In der Regel rückt nur ein einziger Kriminaltechniker/Forensiker an einen Tatort aus. Für die fotografische Dokumentation benötigt er/sie ein Bilderfassungsgerät, beispielsweise ein Handy oder eine Kamera mit Makroobjektiv, einen Massstab und einen gestreckten Finger. Die Handhabung gestaltet sich daher für eine einzige Person mit nur zwei verfügbaren Händen sehr schwierig, wobei der zu dokumentierende Finger während der Leichenstarre teilweise mit erheblichem Kraftaufwand gestreckt und gehalten werden muss. Diese Arbeit erfordert mindestens drei Hände. In einer Hand wird das Bilderfassungsgerät gehalten und fotografiert, die zweite Hand streckt und hält den Finger während der Leichenstarre und eine weitere Hand sollte den Massstab planparallel zu den Papillarleisten halten, um die Dimension des Fingers zu bestätigen.

Weitere gattungsgemässe Vorrichtungen sind in den Dokumenten CN212546942U, EP 1611542 B1 sowie CN109276257 A beschrieben.

### Aufgabe der Erfindung

Daher ist es Aufgabe der Erfindung eine einfachere Vorrichtung zur fotografischen Erfassung der Papillarleisten einer Fingerkuppe einer verstorbenen Person bereitzustellen, insbesondere, wenn die Finger der Person durch eintretende Leichenstarre eine gekrümmte Position einnehmen.

Es ist eine weitere Aufgabe der Erfindung, eine Vorrichtung zur fotografischen Erfassung der Papillarleisten einer Fingerkuppe einer verstorbenen Person bereitzustellen, die von einer einzigen Person bedient werden kann.

### Beschreibung der Erfindung

Die Lösung der Aufgabe der Erfindung erfolgt durch eine Vorrichtung gemäss Anspruch 1. Vorteilhafte Ausführungsbeispiele der Vorrichtung sind Gegenstand der abhängigen Ansprüche.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für die erfindungsgemässe Vorrichtung. Die Beschreibung einer bestimmten Vorrichtung ist nur als beispielhaft anzusehen. In der Beschreibung und den Ansprüchen werden die Begriffe "enthalten", "umfassen", "aufweisen" als "enthalten, aber nicht beschränkt auf" interpretiert.

Eine erfindungsgemässe Vorrichtung zur fotografischen Erfassung der Papillarleisten einer Fingerkuppe einer verstorbenen Person umfasst einen Messkopf, wobei der Messkopf zur Aufnahme eines Fingerendglieds ausgebildet ist, wobei der Messkopf einen Mantel enthält, wobei der Mantel einen ersten Endbereich und einen zweiten Endbereich und eine Mittenachse enthält, wobei der erste Endbereich des Messkopfs als zumindest teilweise geschlossene Oberfläche ausgebildet ist, wobei der zweite Endbereich des Messkopfs als ein Messring ausgebildet ist, wobei ein Messfenster im Mantel des Messkopfs zwischen dem ersten Endbereich und dem zweiten Endbereich angeordnet ist, dadurch gekennzeichnet, dass ein Griffelement sich vom zweiten Endbereich des Messkopfs im Wesentlichen in Richtung der Mittenachse erstreckt. Das Griffelement erleichtert die Überwindung der Leichenstarre durch Unterstützung der Hebelwirkung zur Ausrichtung des Fingers in eine gestreckte Position.

Der Messkopf dient als Positionshalter für die zu dokumentierende Fingerkuppe. Mittels des Messkopfs wird die zentrierte Ausrichtung der Papillarleisten ermöglicht. Insbesondere kann das Messfenster über eine Fensteröffnung verfügen, die von vier Fensterabschnitten umrahmt wird. Zumindest einer der vier Fensterabschnitte kann eine in Millimeterschritten eingeteilte Messskala enthalten. Oberhalb der durch das Messfenster ausgebildeten Fensteröffnung, durch welche ein Sichtfenster ausgebildet ist, befindet sich nach einem Ausführungsbeispiel eine Ringführung für einen Einstellring. Insbesondere kann im ersten Endbereich, welcher den ersten Fensterabschnitt enthält, in dieser Ringführung eine Ausnehmung für den Einsatz eines Magnetelements vorgesehen werden. Vorzugsweise ist die Ausnehmung mittig in Bezug auf den ersten Fensterabschnitt angeordnet. Nach einem exemplarischen Ausführungsbeispiel kann die Ausnehmung einen Innendurchmesser D von beispielsweise 1,5 mm und eine Höhe H von beispielsweise 0,5 mm aufweisen. Das Magnetelement dient zur Positionierung des oder der Anzeigeelemente auf dem Einstellring. Nach einem Ausführungsbeispiel befindet sich im ersten Endbereich zwischen der Ringführung und der Kuppel eine Markierung in Form von einer kleinen Halbkugel, welche die eingestellte Position des Einstellrings bezeichnen soll.

Nach einem Ausführungsbeispiel ist das Griffelement als ein rinnenförmige Auflageelement ausgebildet. In einem als rinnenförmiges Auflageelement ausgebildeten Griffelement kann ein Finger besonders gut positioniert werden. Das rinnenförmige Auflageelement verhindert neben der Materialbeschaffenheit eine Biegung der Vorrichtung. Das rinnenförmige Auflageelement kann insbesondere eine Wölbung aufweisen, die als ein Teil eines Zylinders ausgebildet ist.

Nach einem Ausführungsbeispiel weist das Griffelement eine Länge LG auf, wobei die Länge LG grösser als eine Länge LM des Messkopfs ist, insbesondere mindestens der doppelten Länge LM des Messkopfs entspricht. Mittels eines Griffelements mit einer Länge LG, die grösser als die Länge LM des Messkopfs ist, kann zumindest das mittlere Fingergelenk ebenfalls gestreckt werden, wodurch eine exakte Positionierung des Fingers in der Vorrichtung ermöglicht ist.

Nach einem Ausführungsbeispiel enthält das Griffelement einen Griffelementabschnitt mit einer Einschnürung. Die Einschnürung kann es dem Benutzer erleichtern, das Griffelement zu halten, wenn es nach Einführung des Fingerendglieds in den Messkopf entgegen dem Krümmungsverlauf des Fingers bewegt wird, um den gekrümmten Finger in eine gestreckte Position zu bringen.

Nach einem Ausführungsbeispiel enthält das Griffelement ein Markierungselement. Das Markierungselement kann dem Benutzer ein Referenzmass für die Dicke des zu dokumentierenden, insbesondere fotografierenden, Fingers anzeigen. Dem Benutzer wird hierdurch erleichtert, eine für die Dicke des zu vermessenden Fingers passende Vorrichtung auszuwählen.

Nach einem Ausführungsbeispiel ist der Messkopf ein rotationssymmetrischer Körper ausgebildet ist. Insbesondere kann der Messkopf als ein halbkugelförmiger oder als ein zylinderförmiger Körper ausgebildet sein. Der Messkopf kann insbesondere nach einem Ausführungsbeispiel einen kreisförmigen Querschnitt aufweisen. Ein kreisförmiger Querschnitt ist mit handelsüblichen Werkzeugen einfach herstellbar. Der Messkopf könnte nach einem Ausführungsbeispiel auch einen ovalen Querschnitt aufweisen, um optimal an die Fingerform angepasst zu werden. Selbstverständlich kann die Vorrichtung nach jedem der vorhergehenden Ausführungsbeispiele mittels eines additiven Herstellungsverfahrens hergestellt werden. Insbesondere kann ein selektives Lasersinterverfahren zum Einsatz kommen. Ein selektives Lasersinterverfahren kann insbesondere vorteilhaft zum Einsatz kommen, wenn die Vorrichtung aus einem metallischen Werkstoff besteht.

Nach einem Ausführungsbeispiel ist der Endbereich als eine Kuppel ausgebildet. Insbesondere kann die Kuppel die Form einer Halbkugel aufweisen. Das Innere der Halbkugel ist insbesondere hohl, damit die Fingerspitze des Fingerendglieds im ersten Endbereich genügend Platz finden kann.

Nach einem Ausführungsbeispiel erstreckt sich das Messfenster über einen Winkelbereich des Umfangs des Mantels, der im Bereich von 170 bis einschliesslich 190 Grad liegt. In diesem Winkelbereich ist vorteilhafterweise die gesamte innere Oberfläche der Fingerkuppe sichtbar.

Nach einem Ausführungsbeispiel ist ein Einstellring zwischen dem Messkopf und dem Mantel angeordnet. Insbesondere kann der Mantel ein Deckenelement enthalten.

Nach einem Ausführungsbeispiel kann ein Vorsprung derart auf dem Deckenelement angeordnet sein, dass der Vorsprung dem Messkopf gegenüberliegt. Im zusammengebauten Zustand kann der Messkopf im Vorsprung gehalten werden. Der Messkopf kann hierzu einen Hohlraum enthalten, der eine zum Vorsprung passende Form aufweist, sodass der Messkopf formschlüssig auf dem Vorsprung aufgenommen ist.

Nach einem Ausführungsbeispiel wirkt der Einstellring mit einer Halterung derart zusammen, dass durch den Einstellring und die Halterung im zusammengebauten Zustand eine Ratsche ausgebildet ist. Dieses Ausführungsbeispiel ermöglicht eine besonders einfache Montage. Insbesondere kann die Halterung eine Ausnehmung aufweisen, die zur Aufnahme im Vorsprung ausgebildet ist. Insbesondere können der Vorsprung und die Ausnehmung korrespondierende, insbesondere nicht rotationssymmetrische, Querschnitte aufweisen.

Nach einem Ausführungsbeispiel enthält die Halterung eine Mehrzahl von Armelementen, die sich von einem ersten Endbereich, der mit einem Grundkörper der Halterung verbunden ist, zu einem zweiten Endbereich erstrecken, wobei im zweiten Endbereich jedes Armelements je eine nasenartige Verdickung angeordnet ist, die zum Eingriff in je eine korrespondierende, am Einstellring angeordnete nasenartige Aussparung, ausgebildet ist. Die Armelemente ermöglichen insbesondere eine elastische Verformung und Rückstellung, sodass der Einstellring auf einfache Weise in verschiedene Positionen gedreht werden kann. Der Einstellring kann zumindest eine Anzeigevorrichtung zur Identifikation des Fingers enthalten.

Nach einem Ausführungsbeispiel enthält der Messkopf einen Einstellring. Insbesondere kann der Einstellring im ersten Endbereich angeordnet sein. Der Einstellring kann zumindest eine Anzeigevorrichtung zur Identifikation des Fingers enthalten.

Nach einem Ausführungsbeispiel enthält der Einstellring auf der Innenseite eine Mehrzahl von Ausnehmungen für je ein Magnetelement. Insbesondere kann die Ausnehmung für das Magnetelement oder jede der Ausnehmungen für je ein Magnetelement gegenüberliegend zum entsprechenden Anzeigeelement angeordnet sein. Nach einem Ausführungsbeispiel ist der Einstellring in einer Ringführung aufgenommen, die sich im ersten Endbereich das Messkopfs befindet. In der Ringführung kann eine Ausnehmung für ein Magnetelement vorgesehen sein. Die Ausnehmung in der Ringführung kann insbesondere mittig in Bezug auf den im ersten Endbereich befindlichen ersten Fensterabschnitt angeordnet sein. Wenn ein Magnetelement des Einstellrings über dem Magnetelement in der Ringführung zu liegen kommt, erfolgt eine Anziehung der beiden Magnetelemente, sodass der Einstellring in der gewünschten Einstellung bzw. Position gehalten wird.

Das entsprechende Anzeigeelement zeigt nach jedem der Ausführungsbeispiele die Fingerbezeichnung an. Wenn ein im Messfenster befindlicher Finger fotografisch dokumentiert wird, werden somit nicht nur die an den zweiten, dritten und vierten Fensterabschnitten befindlichen Messskalen, sondern auch die Fingerbezeichnung erfasst.

Somit wird auf der Abbildung der Papillarleisten auf dem Foto auch die Länge und Breite der erfassten Papillarleisten sowie die Information, zu welchem Finger diese Papillarleisten gehören, dokumentiert. Hiermit kann zu einem späteren Zeitpunkt keine Unsicherheit mehr entstehen, zu welchem Finger die fotografisch abgebildete Papillarleiste gehörig ist.

Nach einem Ausführungsbeispiel besteht die Vorrichtung aus einem metallischen Werkstoff, insbesondere aus Edelstahl. Die Verwendung von Edelstahl hat den Vorteil, dass die Vorrichtung nach dem Gebrauch ohne Probleme von Hand oder maschinell gereinigt und wiederverwendet werden kann. Besonders vorteilhaft kann ein Edelstahl mit der Typenbezeichnung 1.4404 zum Einsatz kommen.

Nach einem Ausführungsbeispiel ist die Vorrichtung als ein Einwegartikel konzipiert. Ein Einwegartikel hat den Vorteil, dass eine Reinigung der Vorrichtung nicht erforderlich ist, da sie nur zum einmaligen Gebrauch bestimmt ist. Insbesondre kann die Vorrichtung zumindest einen Kunststoff enthalten oder aus zumindest einem Kunststoff bestehen.

Die erfindungsgemässe Vorrichtung kann, wie nachfolgend beschrieben, verwendet werden: In einem ersten Schritt wird eine zu dem zu dokumentierenden Finger der verstorbenen Person passende Vorrichtung ausgewählt. Danach kann der zu dokumentierende Finger mit der zweiten Hand etwas aus der geballten Faust gezogen werden, um das Fingerendglied des Fingers in den Messkopf der Vorrichtung zu legen, sodass das Fingerendglied bis zum ersten Endbereich eindringt, der einen Anschlag für die Fingerspitze des Fingerendglieds ausbildet. Die Papillarleistenseite des Fingers ist hierbei in Richtung der Fensteröffnung des Messfensters der Vorrichtung ausgerichtet.

Nun wird das Griffstück mit einer Hebelbewegung zum Handrücken bewegt. Durch die entstandene Hebelwirkung wird der Finger mühelos gestreckt. Im selben Prozess wird die benötigte Papillarleiste des Fingerendgliedes in der bemassten Fensteröffnung des Messkopfs sichtbar. Der Einstellring kann nun auf die entsprechende Fingerbezeichnung durch Drehen eingestellt werden (linker Zeigefinger = F7).

Nun kann die eine Hand für das Halten der Vorrichtung am Handrücken der Leiche eingesetzt werden, während der Anwender mit der zweiten Hand das Bilderfassungsgerät hält und die fotografische Dokumentation durchführt. Das Fenster erlaubt aufgrund des grosszügigen Ausschnitts auch eine fotografische Dokumentation der seitlich gelegenen Papillarleisten zu erstellen.

Wenn dem Eintritt der Leichenstarre eine Identifizierung der verstorbenen Person durch die Papillarleisten der Finger erforderlich ist, kann ein allein arbeitender Kriminaltechniker oder Forensiker mit herkömmlichen Vorrichtungen oder Methoden die Identifizierung sowie die hierzu erforderliche Dokumentation nur erschwert vornehmen. Das herkömmliche Verfahren zur Erstellung einer Dokumentation der Fingerpapillarleisten erfordert oft mehr als zwei Hände und ist daher ineffizient, weil für eine effiziente Dokumentation die Hilfe einer weiteren Person in Anspruch genommen werden müsste. Die erfindungsgemässe Vorrichtung ermöglicht es, den Finger zu strecken, die Papillarleisten sichtbar zu machen und gleichzeitig mit einem planparallelen Massstab zu fotografieren. Die Qualität der Dokumentation wird somit mittels der erfindungsgemässen Vorrichtung verbessert, weil eine saubere Darstellung der Papillarleisten erfolgen kann. Insbesondere kommt es nicht zum Verzug der Papillarleisten bedingt durch das Strecken der Finger der verstorbenen Person mit blossen Händen.

### Kurzbeschreibung der Zeichnungen

Nachfolgend wird die erfindungsgemässe Vorrichtung anhand einiger Ausführungsbeispiele dargestellt. Es zeigen
Fig. 1a eine seitliche Ansicht eines ersten Ausführungsbeispiels der Vorrichtung,
Fig. 1b eine Ansicht der Vorrichtung gemäss Fig. 1a von vorne,
Fig. 1c eine Ansicht der Vorrichtung gemäss Fig. 1a von unten,
Fig. 1d eine räumliche Ansicht der Vorrichtung gemäss Fig. 1a,
Fig. 2a eine seitliche Ansicht eines zweiten Ausführungsbeispiels der Vorrichtung,
Fig. 2b eine Ansicht der Vorrichtung gemäss Fig. 2a von vorne,
Fig. 2c eine Ansicht der Vorrichtung gemäss Fig. 2a von unten,
Fig. 2d eine räumliche Ansicht der Vorrichtung gemäss Fig. 2a,
Fig. 3a ein Detail eines Einstellrings in einer räumlichen Darstellung,
Fig. 3b eine Ansicht des Einstellrings gemäss Fig. 3a von unten,
Fig. 3c eine Ansicht des Einstellrings gemäss Fig. 3a von vorne,
Fig. 4a eine seitliche Ansicht eines dritten Ausführungsbeispiels der Vorrichtung,
Fig. 4b eine Ansicht der Vorrichtung gemäss Fig. 4a von vorne,
Fig. 4c eine räumliche Ansicht der Vorrichtung gemäss Fig. 4a,
Fig. 4d eine weitere räumliche Ansicht der Vorrichtung gemäss Fig. 4a,
Fig. 4e ein Detail der Fig. 4c,
Fig. 4f ein Detail der Fig. 4d,
Fig. 4g ein Detail eines Einstellrings und einer Halterung für eine Vorrichtung nach Fig. 4a-4f,
Fig. 4h ein Detail des Einstellrings und der Halterung für eine Vorrichtung nach Fig. 4a-4f in einer Explosionsdarstellung.

### Detaillierte Beschreibung der Zeichnungen

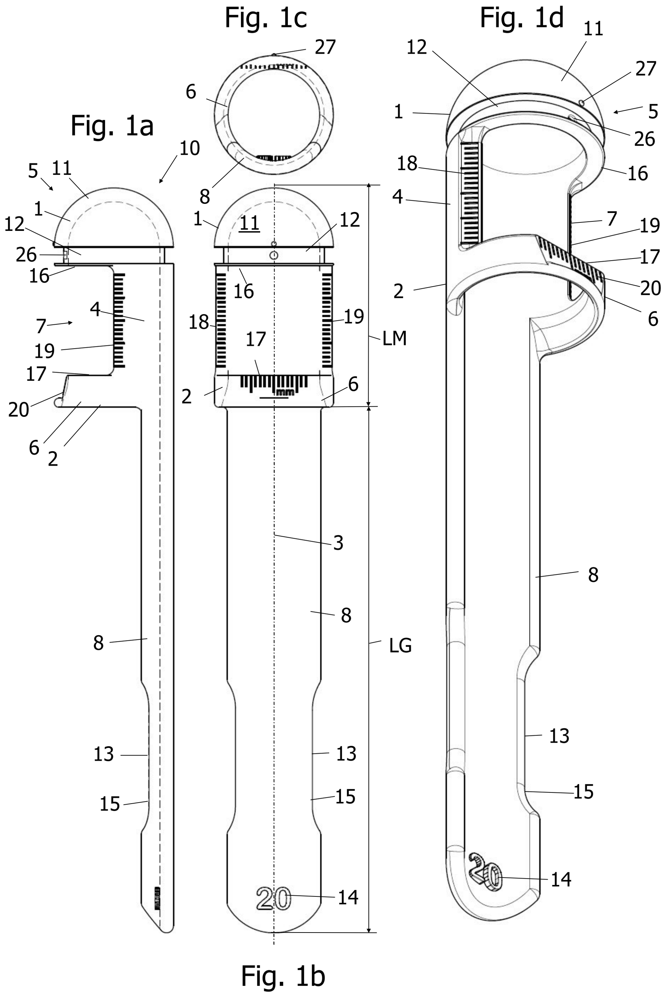

Fig. 1a bis 1d zeigen eine Vorrichtung 10 zur fotografischen Erfassung der Papillarleisten einer Fingerkuppe einer verstorbenen Person umfassend einen Messkopf 5 nach einem ersten Ausführungsbeispiel. Insbesondere zeigt Fig. 1a eine seitliche Ansicht eines ersten Ausführungsbeispiels der erfindungsgemässen Vorrichtung 10, Fig. 1b eine Ansicht der Vorrichtung gemäss Fig. 1a von vorne, Fig. 1c eine Ansicht der Vorrichtung gemäss Fig. 1a von unten und Fig. 1d eine räumliche Ansicht der Vorrichtung gemäss Fig. 1a.

Der Messkopf 5 ist zur Aufnahme eines Fingerendglieds ausgebildet. Der Messkopf 5 enthält einen Mantel 4, wobei der Mantel 4 einen ersten Endbereich 1 und einen zweiten Endbereich 2 und eine Mittenachse 3 enthält. Der erste Endbereich 1 des Messkopfs 5 ist als zumindest teilweise geschlossene Oberfläche ausgebildet. Durch den ersten Endbereich 1 kann insbesondere ein Anschlag für eine Fingerspitze eines Fingerendglieds eines mit der Vorrichtung zu identifizierenden Fingers ausgebildet sein. Der zweite Endbereich 2 des Messkopfs 5 umfasst einen Messring 6. Ein Messfenster 7 ist im Mantel 4 des Messkopfs 5 zwischen dem ersten Endbereich 1 und dem zweiten Endbereich 2 angeordnet. Ein Griffelement 8 erstreckt sich vom zweiten Endbereich 2 des Messkopfs 5 im Wesentlichen in Richtung der Mittenachse 3. In Markierungselment 14 kann im Bereich der Mittenachse angeordnet sein. Das Markierungselement 14 enthält nach einem Ausführungsbeispiel eine Grössenbezeichnung für einen Finger.

Der Messkopf 5 dient als Positionshalter des zu dokumentierenden Fingerendgliedes. Mittels des Messkopfs 5 wird die zentrierte Ausrichtung der Papillarleisten eines Fingerendgliedes eines zu dokumentierenden Fingers ermöglicht. Insbesondere kann das Messfenster 7 über eine Fensteröffnung verfügen, die von einem ersten, einem zweiten, einem dritten und einem vierten Fensterabschnitt 16, 17, 18, 19 umrahmt wird. Zumindest einer der Fensterabschnitte kann eine in Millimeterschritten eingeteilte Messskala enthalten, im vorliegenden Ausführungsbeispiel enthalten die zweiten, dritten und vierten Fensterabschnitte 17, 18, 19 je eine Messskala. Oberhalb der Fensteröffnung, durch welche ein Sichtfenster ausgebildet ist, befindet sich nach einem Ausführungsbeispiel eine Ringführung 12 für einen Einstellring. Insbesondere kann im Bereich des ersten Fensterabschnitts 16 in dieser Ringführung 12 eine Ausnehmung 26 für den Einsatz eines Magnetelements vorgesehen werden. Nach einem exemplarischen Ausführungsbeispiel kann die Ausnehmung 26 einen Innendurchmesser D von 1,5 mm und eine Höhe H von 0,5 mm aufweisen. Das Magnetelement dient zur Positionierung des oder der Anzeigeelemente 28 auf dem Einstellring 9. In der vorliegenden Darstellung befindet sich oberhalb der Ringführung 12 für den Einstellring 9 eine Markierung 27 in Form von einer kleinen Halbkugel, welche die eingestellte Position des Einstellrings 9 bezeichnet.

Nach dem vorliegenden Ausführungsbeispiel ist das Griffelement 8 als ein rinnenförmiges Auflageelement ausgebildet, welches zur Auflage eines gestreckten Fingers ausgebildet ist.

Nach dem vorliegenden Ausführungsbeispiel weist das Griffelement 8 eine Länge LG auf, wobei die Länge LG grösser als eine Länge LM des Messkopfs 5 ist. Insbesondere kann die Länge LG mindestens der doppelten Länge LM des Messkopfs 5 entsprechen.

Nach einem Ausführungsbeispiel enthält das Griffelement 8 einen Griffelementabschnitt 13 mit einer Einschnürung 15. Die Einschnürung 15 kann es dem Benutzer erleichtern, dass Griffelement 8 zu halten, wenn es nach Einführung des Fingerendglieds in den Messkopf 5 entgegen dem Krümmungsverlauf des Fingers bewegt wird, um den gekrümmten Finger in eine gestreckte Position zu bringen.

Nach dem vorliegenden Ausführungsbeispiel enthält das Griffelement 8 ein Markierungselement 14. Das Markierungselement 14 kann dem Benutzer ein Referenzmass für die Dicke des zu vermessenden Fingers anzeigen. Dem Benutzer wird hierdurch erleichtert, eine für die Dicke des zu vermessenden Fingers passende Vorrichtung aus einem Set von Vorrichtungen für unterschiedliche Fingerdicken auszuwählen.

Nach dem vorliegenden Ausführungsbeispiel ist der Messkopf 5 als ein zylinderförmiger Körper ausgebildet. Der Messkopf 5 hat nach dem vorliegenden Ausführungsbeispiel einen kreisförmigen Querschnitt. Ein kreisförmiger Querschnitt ist mit handelsüblichen Werkzeugen einfach herstellbar. Der Messkopf 5 könnte nach einem nicht dargestellten Ausführungsbeispiel auch einen ovalen Querschnitt aufweisen, um optimal an die Fingerform angepasst zu werden. Selbstverständlich kann die Vorrichtung nach jedem der vorhergehenden Ausführungsbeispiele auch mittels eines additiven Herstellungsverfahrens hergestellt werden.

Nach dem vorliegenden Ausführungsbeispiel ist der erste Endbereich 1 als eine Kuppel 11 ausgebildet. Die Kuppel 11 weist nach dem vorliegenden Ausführungsbeispiel eine Form einer Halbkugel auf. Das Innere der Halbkugel ist hohl, damit die Fingerspitze des Fingers in der Kuppel 11 des ersten Endbereichs 1 genügend Platz finden kann. Nach einem nicht dargestellten Ausführungsbeispiel kann der erste Endbereich 1 auch zumindest abschnittsweise kegelförmig ausgebildet sein. Nach einem weiteren Ausführungsbeispiel kann der erste Endabschnitt eine Öffnung aufweisen. Die Öffnung ist kleiner als die Querschnittsfläche des Innenraums des Mantelbereichs, sodass die Fingerspitze in der Öffnung gehalten werden kann. Wenn eine Öffnung im ersten Endbereich 1 vorgesehen ist, kann sich eine Reinigung des Innenraums des ersten Endbereichs als besonders einfach erweisen. Der Innenraum des ersten Endbereichs kann einfach durchspült werden. Die Öffnung ist somit als Spülöffnung ausgebildet.

Nach dem vorliegenden Ausführungsbeispiel erstreckt sich das Messfenster 7 über einen Winkelbereich eines Umfangs des Mantels 4, der im Bereich von 170 bis einschliesslich 190 Grad liegt. Im Messfenster 7 ist somit die gesamte innere Oberfläche der Fingerkuppe sichtbar. Insbesondere können auch seitlich liegende Papillarleisten gut abgebildet werden. Das Messfenster 7 wird durch einen ersten Fensterabschnitt 16 begrenzt, welcher durch das Ende des ersten Endbereichs 1 ausgebildet ist. Gegenüberliegend zum ersten Fensterabschnitt 16 befindet sich ein zweiter Fensterabschnitt 17. Der zweite Fensterabschnitt 17 ist insbesondere am Messring 6 angeordnet. Ein dritter Fensterabschnitt 18 und ein vierter Fensterabschnitt 19 erstrecken sich vom ersten Fensterabschnitt 16 zum zweiten Fensterabschnitt 17 und bilden die seitliche Begrenzung des Messfensters 7 aus. Mit einer seitlichen Begrenzung ist im vorliegenden Ausführungsbeispiel eine Begrenzung gemeint, die parallel zur Mittenachse 3 verläuft, sie kann auch als axiale Begrenzung des Fensters 7 bezeichnet werden. Der erste Fensterabschnitt 16 und der zweite Fensterabschnitt 17 verlaufen nach dem vorliegenden Ausführungsbeispiel in Normalebenen zur Mittenachse 3.

Der zweite Fensterabschnitt 17 enthält nach dem vorliegenden Ausführungsbeispiel eine Messskala. Beispielsweise kann die Messskala eine Länge in mm angegeben werden. Die Messskala ist nach dem vorliegenden Ausführungsbeispiel in einem ebenen Abschnittsbereich 20 des zweiten Endbereichs 2 angeordnet, welcher den Messring 6 ausbildet.

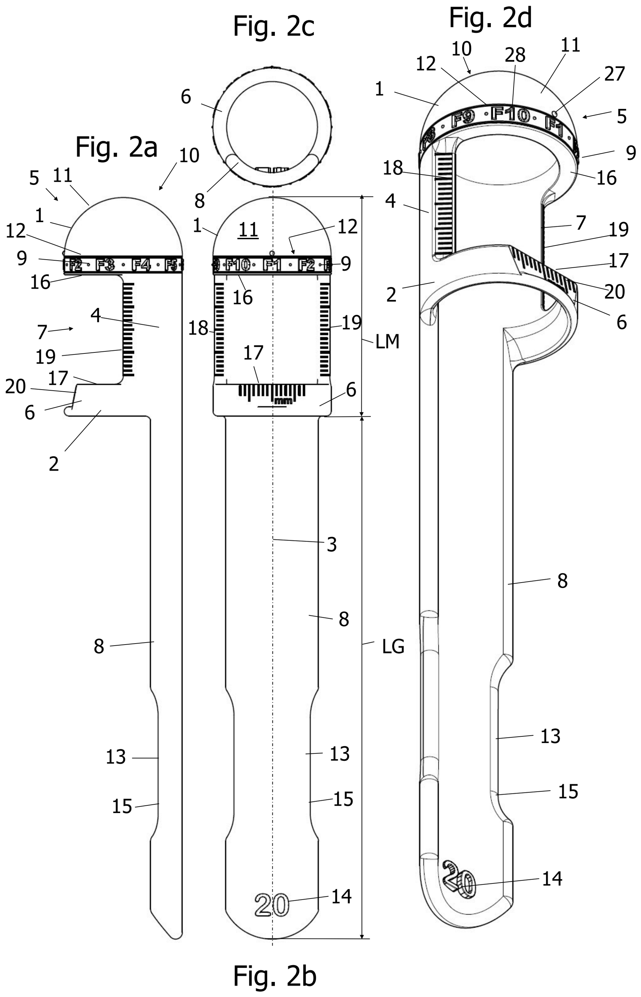

Fig. 2a bis 2d zeigen eine Vorrichtung 10 zur fotografischen Erfassung der Papillarleisten einer Fingerkuppe einer verstorbenen Person umfassend einen Messkopf 5 nach einem zweiten Ausführungsbeispiel. Für gleiche oder gleichwirkende Bauelemente wurden dieselben Bezugszeichen wie im ersten Ausführungsbeispiel verwendet. Insbesondere zeigt Fig. 2a eine seitliche Ansicht eines ersten Ausführungsbeispiels der erfindungsgemässen Vorrichtung 10, Fig. 2b eine Ansicht der Vorrichtung gemäss Fig. 2a von vorne, Fig. 2c eine Ansicht der Vorrichtung gemäss Fig. 2a von unten und Fig. 2d eine räumliche Ansicht der Vorrichtung gemäss Fig. 2a. Auf die Beschreibung zu Fig. 1a bis 1d wird an dieser Stelle verwiesen.

Nach dem vorliegenden Ausführungsbeispiel enthält der Messkopf 5 einen Einstellring 9. Insbesondere kann der Einstellring 9 im ersten Endbereich 1 angeordnet sein. Der Einstellring 9 ist relativ zum ersten Endbereich 1 drehbar. Der Einstellring 9 kann zumindest ein Anzeigeelement 28 zur Identifikation des Fingers enthalten. Nach dem vorliegenden Ausführungsbeispiel ist der Einstellring 9 in einer Ringführung 12 aufgenommen, die sich im ersten Endbereich 1 des Messkopfs 5 befindet. In der Ringführung 12 kann eine Ausnehmung 26 für ein Magnetelement vorgesehen sein, sie ist in Fig. 1d gezeigt. Die Ausnehmung 26 in der Ringführung 12 kann insbesondere mittig in Bezug auf den im ersten Endbereich befindlichen ersten Fensterabschnitt angeordnet sein. Wenn ein Magnetelement des Einstellrings 9 über dem Magnetelement in der Ringführung 12 zu liegen kommt, erfolgt eine Anziehung der beiden gegenüberliegenden Magnetelemente, sodass der Einstellring 9 in der gewünschten Einstellung gehalten wird. Das entsprechende Anzeigeelement 28 zeigt die Fingerbezeichnung an.

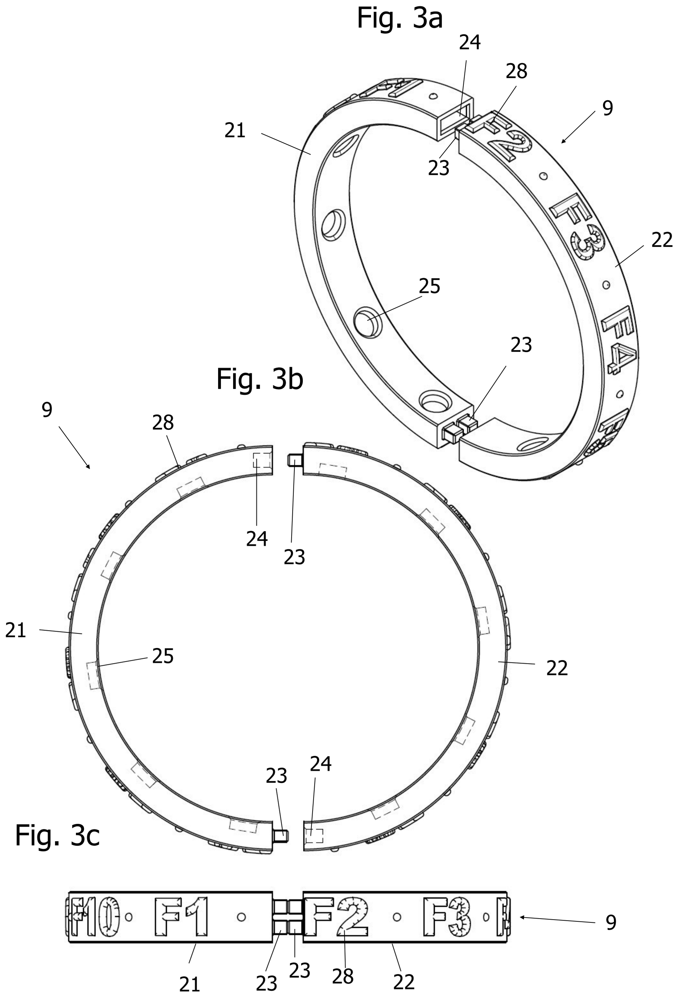

Fig. 3a zeigt ein Detail eines Einstellrings 9 nach dem in Fig. 2a bis Fig. 2d gezeigten zweiten Ausführungsbeispiel in einer räumlichen Darstellung. Fig. 3b zeigt eine Ansicht des Einstellrings 9 gemäss Fig. 3a von unten und Fig. 3c zeigt eine Ansicht des Einstellrings 9 gemäss Fig. 3a von vorne. Nach dem vorliegenden Ausführungsbeispiel enthält der Einstellring 9 ein erstes Einstellringteilelement 21 und ein zweites Einstellringteilelement 22.

Jedes der ersten und zweiten Einstellringteilelemente 21, 22 enthält seinem Ende nach dem vorliegenden Ausführungsbeispiel ein Teilelement einer Steckverbindung. Nach dem vorliegenden Ausführungsbeispiel enthält eines der ersten oder zweiten Enden des ersten oder zweiten Einstellringteilelements 21, 22 zumindest ein Stiftelement 23, welches im zusammengebauten Zustand in einer entsprechenden Ausnehmung 24 des korrespondierenden zweiten oder ersten Einstellringteilelements 22, 21 aufgenommen ist.

Auf der Innenseite des ersten und zweiten Einstellringteilelements 21, 22 sind eine Mehrzahl von Ausnehmungen 25 für Magnetelemente angebracht. Jede der Ausnehmungen 25 kann ein knopfzellenartiges Magnetelement aufnehmen, welches zeichnerisch nicht dargestellt ist. In der einfachsten Ausführung ist das Magnetelement als ein Zylinder ausgebildet, welcher in der Ausnehmung 25 aufgenommen werden kann. Wenn der erste Endbereich 1 ein magnetisches oder magnetisierbares Element in der Ringführung 12 enthält, welche zur Aufnahme des Einstellrings 9 ausgebildet ist, kann der Einstellring 9 an der gewünschten Position gehalten werden, um die korrekte Bezeichnung des in der Vorrichtung befindlichen Fingers anzuzeigen.

Insbesondere besteht der Einstellring 9 aus zwei Einstellringteilelementen 21, 22, die als Halbringe ausgebildet sind, und ist innenseitig mit zumindest einer Ausnehmung 25 für zumindest ein Magnetelement oder eine entsprechende Anzahl Magnetelemente versehen, wenn mehrere Ausnehmungen 25 vorgesehen sind, wie in Fig. 3a bis Fig. 3c gezeigt. Nach einem exemplarischen Ausführungsbeispiel kann die Ausnehmung 25 bzw. jede der Ausnehmungen 25 als eine Sacklochbohrung ausgebildet sein. Die Ausnehmung 25 kann nach einem exemplarischen Ausführungsbeispiel einen Innendurchmesser D von 1,5 mm und eine Höhe H von 0,5 mm aufweisen. Wenn eine Mehrzahl von Ausnehmungen 25 vorgesehen ist, können die Ausnehmungen 25 vorzugsweise in gleichen Abständen zueinander angeordnet sein. An der Aussenseite des Einstellrings 9 ist zumindest ein Anzeigeelement 28 angeordnet. Insbesondere kann eine Mehrzahl von Anzeigeelementen 28 vorgesehen werden. Beispielsweise können die Anzeigeelemente 28 die international standardisierten Fingerbezeichnungen enthalten.

| | | |
|---|---|---|
| F1: | rechter | Daumen |
| F2: | rechter | Zeigefinger |
| F3: | rechter | Mittelfinger |
| F4: | rechter | Ringfinger |
| F5: | rechter | Kleinfinger |
| F6: | linker | Daumen |
| F7: | linker | Zeigefinger |
| F8: | linker | Mittelfinger |
| F9: | linker | Ringfinger |
| F10: | linker | Kleinfinger |

Daher sind nach einem bevorzugten Ausführungsbeispiel zehn Anzeigeelemente 28 vorgesehen. Die Anzahl der Ausnehmungen 25 kann insbesondere der Anzahl der Anzeigeelemente 28 entsprechen. Daher sind bevorzugt zehn Ausnehmungen 25 für zehn zugehörige Magnetelemente vorgesehen.

Die ersten und zweiten Einstellringteilelemente 21, 22 können in der Ringführung 12 des Messkopfs 5 zu dem Einstellring 9 zusammengefügt werden. Der Einstellring 9 lässt sich in der Ringführung 12 drehen, um das gewünschte Anzeigeelement 28 unterhalb der Markierung 27 zu setzen. Das Magnetelement des Messkopfs 5 sowie das Magnetelement des eingestellten Anzeigeelements 28 auf dem Einstellring 9 ziehen sich dabei an und halten den Einstellring 9 in der gewünschten Position, beispielsweise für die gewünschte Fingerbezeichnung. Mit der eingestellten Fingerbezeichnung kann die Fingerposition der rechten oder linken Hand später dem fotografisch ermittelten Abbild der Papillarleisten unmittelbar und eindeutig zugeordnet werden.

Die Vorrichtung nach jedem der vorhergehenden Ausführungsbeispiele kann insbesondere als Set in verschiedenen Abmessungen bereitgestellt werden.

Beispielhaft können folgende Abmessungen der Vorrichtung verwendet werden:

| | |
|---|---|
| Variante 1: | Durchmesser: Innen 18 mm / Aussen 24 mm, Gesamtlänge 150 mm |
| Variante 2: | Durchmesser: Innen 20 mm / Aussen 26 mm, Gesamtlänge 150 mm |
| Variante 3: | Durchmesser: Innen 22 mm / Aussen 28 mm, Gesamtlänge 150 mm |
| Variante 4: | Durchmesser: Innen 24 mm / Aussen 30 mm, Gesamtlänge 150 mm |

Mit dem Durchmesser ist hierbei der Innendurchmesser eines Messkopfs 5 gemeint, der einen kreisförmigen Querschnitt aufweist. Die Gesamtlänge entspricht nach diesem exemplarischen Ausführungsbeispiel der Summe der Länge LG und der Länge LM. Mit einem Set, welches Variante 1, Variante 2, Variante 3 und Variante 4 umfasst, kann ein Grossteil der vorkommenden Fingerdicken abgedeckt werden.

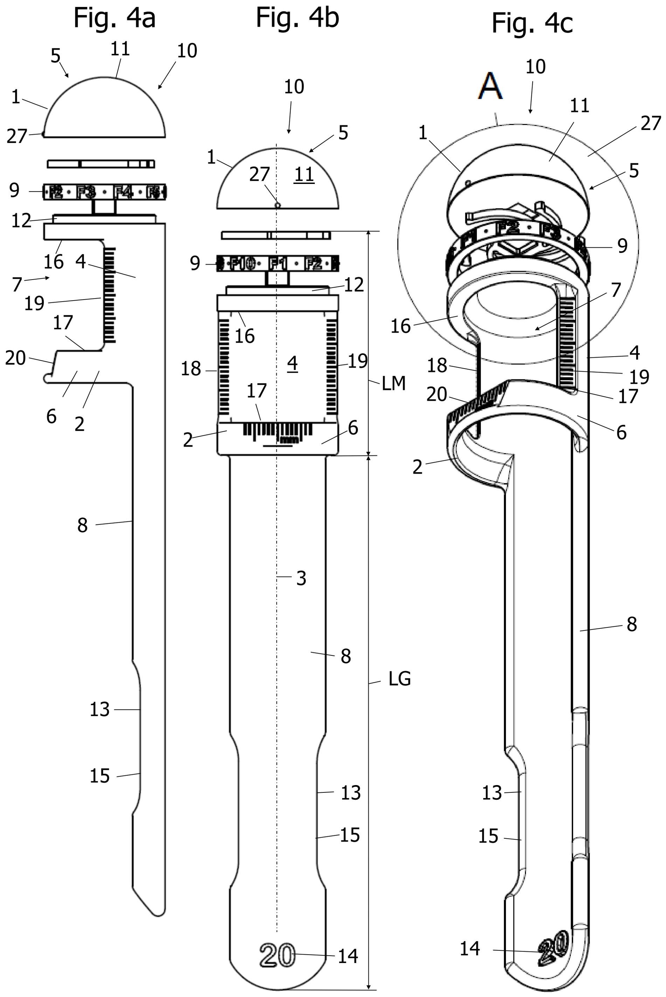

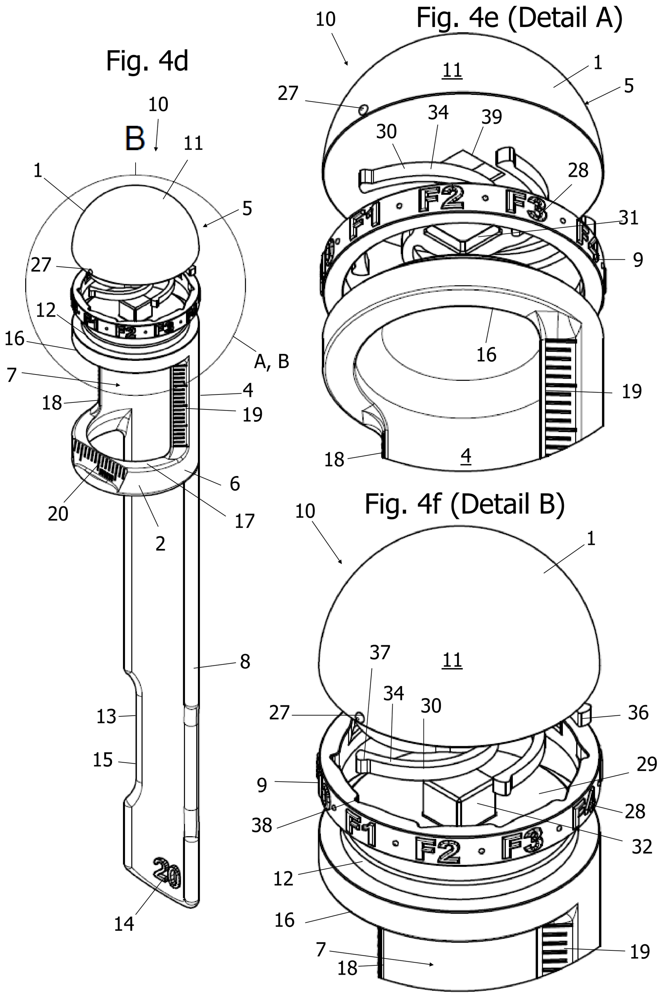

Fig. 4a bis 4f zeigen eine Vorrichtung 10 zur fotografischen Erfassung der Papillarleisten einer Fingerkuppe einer verstorbenen Person umfassend einen Messkopf 5 nach einem dritten Ausführungsbeispiel. Für gleiche oder gleichwirkende Bauelemente wurden dieselben Bezugszeichen wie im ersten Ausführungsbeispiel verwendet. Insbesondere zeigt Fig. 4a eine seitliche Ansicht eines dritten Ausführungsbeispiels der erfindungsgemässen Vorrichtung 10, Fig. 4b eine Ansicht der Vorrichtung gemäss Fig. 4a von vorne, Fig. 4c eine räumliche Ansicht der Vorrichtung gemäss Fig. 4a und Fig. 4d eine weitere räumliche Ansicht der Vorrichtung gemäss Fig. 4a.

Der Messkopf 5 ist zur Aufnahme eines Fingerendglieds ausgebildet. Der Messkopf 5 enthält einen Mantel 4, wobei der Mantel 4 einen ersten Endbereich 1 und einen zweiten Endbereich 2 und eine Mittenachse 3 enthält. Der erste Endbereich 1 des Messkopfs 5 ist als zumindest teilweise geschlossene Oberfläche ausgebildet. Durch den ersten Endbereich 1 kann insbesondere ein Anschlag für eine Fingerspitze eines Fingerendglieds eines mit der Vorrichtung zu identifizierenden Fingers ausgebildet sein. Der zweite Endbereich 2 des Messkopfs 5 umfasst einen Messring 6. Ein Messfenster 7 ist im Mantel 4 des Messkopfs 5 zwischen dem ersten Endbereich 1 und dem zweiten Endbereich 2 angeordnet. Ein Griffelement 8 erstreckt sich vom zweiten Endbereich 2 des Messkopfs 5 im Wesentlichen in Richtung der Mittenachse 3.

Der Messkopf 5 dient als Positionshalter des zu dokumentierenden Fingerendgliedes. Mittels des Messkopfs 5 wird die zentrierte Ausrichtung der Papillarleisten eines Fingerendgliedes eines zu dokumentierenden Fingers ermöglicht. Insbesondere kann das Messfenster 7 über eine Fensteröffnung verfügen, die von einem ersten, einem zweiten, einem dritten und einem vierten Fensterabschnitt 16, 17, 18, 19 umrahmt wird. Zumindest einer der Fensterabschnitte kann eine in Millimeterschritten eingeteilte Messskala enthalten, im vorliegenden Ausführungsbeispiel enthalten die zweiten, dritten und vierten Fensterabschnitte 17, 18, 19 je eine Messskala. Oberhalb der Fensteröffnung, durch welche ein Sichtfenster ausgebildet ist, befindet sich nach einem Ausführungsbeispiel eine Ringführung 12 für einen Einstellring 9.

In der vorliegenden Darstellung befindet sich oberhalb der Ringführung 12 für den Einstellring 9 eine Markierung 27 in Form von einer kleinen Halbkugel, welche die eingestellte Position des Einstellrings 9 bezeichnet.

Nach dem vorliegenden Ausführungsbeispiel ist das Griffelement 8 als ein rinnenförmiges Auflageelement ausgebildet, welches zur Auflage eines gestreckten Fingers ausgebildet ist.

Nach dem vorliegenden Ausführungsbeispiel weist das Griffelement 8 eine Länge LG auf, wobei die Länge LG grösser als eine Länge LM des Messkopfs 5 ist. Insbesondere kann die Länge LG mindestens der doppelten Länge LM des Messkopfs 5 entsprechen.

Nach einem Ausführungsbeispiel enthält das Griffelement 8 einen Griffelementabschnitt 13 mit einer Einschnürung 15. Die Einschnürung 15 kann es dem Benutzer erleichtern, dass Griffelement 8 zu halten, wenn es nach Einführung des Fingerendglieds in den Messkopf 5 entgegen dem Krümmungsverlauf des Fingers bewegt wird, um den gekrümmten Finger in eine gestreckte Position zu bringen.

Nach dem vorliegenden Ausführungsbeispiel enthält das Griffelement 8 ein Markierungselement 14. Das Markierungselement 14 kann dem Benutzer ein Referenzmass für die Dicke des zu vermessenden Fingers anzeigen. Dem Benutzer wird hierdurch erleichtert, eine für die Dicke des zu vermessenden Fingers passende Vorrichtung aus einem Set von Vorrichtungen für unterschiedliche Fingerdicken auszuwählen.

Nach dem vorliegenden Ausführungsbeispiel ist der Messkopf 5 als ein zylinderförmiger Körper ausgebildet. Der Messkopf 5 hat nach dem vorliegenden Ausführungsbeispiel einen kreisförmigen Querschnitt. Ein kreisförmiger Querschnitt ist mit handelsüblichen Werkzeugen einfach herstellbar. Der Messkopf 5 könnte nach einem nicht dargestellten Ausführungsbeispiel auch einen ovalen Querschnitt aufweisen, um optimal an die Fingerform angepasst zu werden. Selbstverständlich kann die Vorrichtung nach jedem der vorhergehenden Ausführungsbeispiele auch mittels eines additiven Herstellungsverfahrens hergestellt werden.

Nach dem vorliegenden Ausführungsbeispiel ist der erste Endbereich 1 als eine Kuppel 11 ausgebildet. Die Kuppel 11 weist nach dem vorliegenden Ausführungsbeispiel eine Form einer Halbkugel auf. Das Innere der Halbkugel ist hohl, damit die Fingerspitze des Fingers in der Kuppel 11 des ersten Endbereichs 1 genügend Platz finden kann. Nach einem nicht dargestellten Ausführungsbeispiel kann der erste Endbereich 1 auch zumindest abschnittsweise kegelförmig ausgebildet sein. Nach einem weiteren Ausführungsbeispiel kann der erste Endabschnitt eine Öffnung aufweisen. Die Öffnung ist kleiner als die Querschnittsfläche des Innenraums des Mantelbereichs, sodass die Fingerspitze in der Öffnung gehalten werden kann. Wenn eine Öffnung im ersten Endbereich 1 vorgesehen ist, kann sich eine Reinigung des Innenraums des ersten Endbereichs als besonders einfach erweisen. Der Innenraum des ersten Endbereichs kann einfach durchspült werden. Die Öffnung ist somit nach einem nicht dargestellten Ausführungsbeispiel als Spülöffnung ausgebildet.

Nach dem vorliegenden Ausführungsbeispiel erstreckt sich das Messfenster 7 über einen Winkelbereich eines Umfangs des Mantels 4, der im Bereich von 170 bis einschliesslich 190 Grad liegt. Im Messfenster 7 ist somit die gesamte innere Oberfläche der Fingerkuppe sichtbar. Insbesondere können auch seitlich liegende Papillarleisten gut abgebildet werden. Das Messfenster 7 wird durch einen ersten Fensterabschnitt 16 begrenzt, welcher durch das Ende des ersten Endbereichs 1 ausgebildet ist. Gegenüberliegend zum ersten Fensterabschnitt 16 befindet sich ein zweiter Fensterabschnitt 17. Der zweite Fensterabschnitt 17 ist insbesondere am Messring 6 angeordnet. Ein dritter Fensterabschnitt 18 und ein vierter Fensterabschnitt 19 erstrecken sich vom ersten Fensterabschnitt 16 zum zweiten Fensterabschnitt 17 und bilden die seitliche Begrenzung des Messfensters 7 aus. Mit einer seitlichen Begrenzung ist im vorliegenden Ausführungsbeispiel eine Begrenzung gemeint, die parallel zur Mittenachse 3 verläuft, sie kann auch als axiale Begrenzung des Fensters 7 bezeichnet werden. Der erste Fensterabschnitt 16 und der zweite Fensterabschnitt 17 verlaufen nach dem vorliegenden Ausführungsbeispiel in Normalebenen zur Mittenachse 3.

Der zweite Fensterabschnitt 17 enthält nach dem vorliegenden Ausführungsbeispiel eine Messskala. Beispielsweise kann die Messskala eine Länge in mm angegeben werden. Die Messskala ist nach dem vorliegenden Ausführungsbeispiel in einem ebenen Abschnittsbereich 20 des zweiten Endbereichs 2 angeordnet, welcher den Messring 6 ausbildet.

Fig. 4e zeigt das Detail A von Fig. 4c und Fig. 4f das Detail B von Fig. 4d. Nach diesem Ausführungsbeispiel ist ein Einstellring 9 zwischen dem Messkopf 5 und dem Mantel 4 angeordnet. Insbesondere kann der Mantel ein Deckenelement 29 enthalten. Ein Vorsprung 32 ist insbesondere derart auf dem Deckenelement 29 angeordnet, dass der Vorsprung 32 dem Messkopf 5 gegenüberliegt. Im zusammengebauten Zustand kann der Messkopf 5 im Vorsprung 32 gehalten werden. Der Messkopf 5 kann hierzu einen Hohlraum 39 enthalten, der eine zum Vorsprung 32 passende Form aufweist, sodass der Messkopf 5 im zusammengebauten Zustand formschlüssig auf dem Vorsprung 32 aufgenommen ist.

Nach dem vorliegenden Ausführungsbeispiel wirkt der Einstellring 9 mit einer Halterung 30 derart zusammen, dass durch den Einstellring 9 und die Halterung 30 im zusammengebauten Zustand eine Ratsche ausgebildet ist. Dieses Ausführungsbeispiel ermöglicht eine besonders einfache Montage. Insbesondere kann die Halterung 30 eine Ausnehmung 31 aufweisen, die zur Aufnahme im Vorsprung 32 ausgebildet ist. Insbesondere können der Vorsprung 32 und die Ausnehmung 31 korrespondierende, insbesondere nicht rotationssymmetrische, Querschnitte aufweisen.

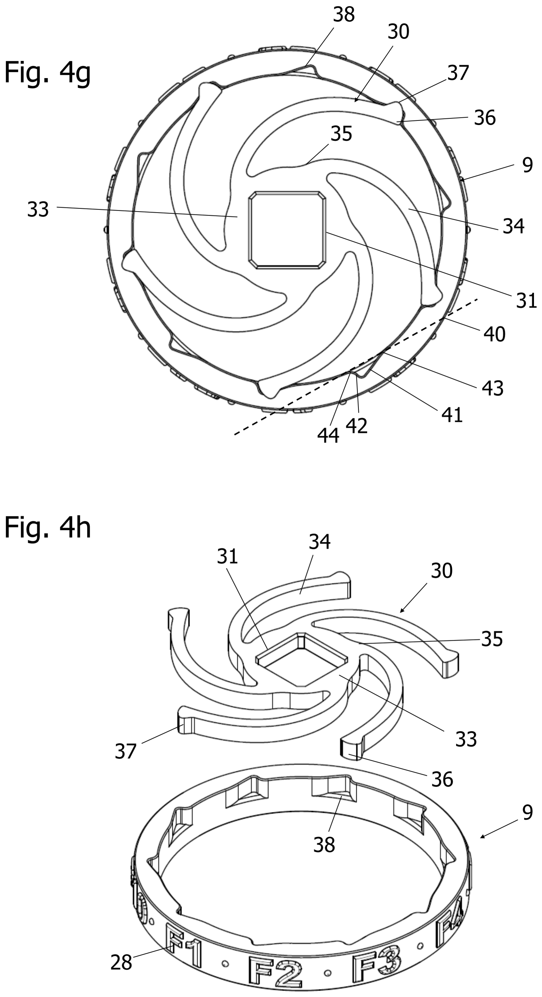

Nach dem vorliegenden Ausführungsbeispiel enthält die Halterung 30 eine Mehrzahl von Armelementen 34, die sich von einem ersten Endbereich 35, der mit einem Grundkörper 33 der Halterung 30 verbunden ist, zu einem zweiten Endbereich 36 erstrecken, was in Fig. 4g und Fig. 4h gezeigt ist. Im zweiten Endbereich 36 jedes Armelements 34 ist je eine nasenartige Verdickung 37 angeordnet, die zum Eingriff in je eine korrespondierende, am Einstellring 9 angeordnete nasenartige Aussparung 38 ausgebildet ist. Die Armelemente 34 ermöglichen insbesondere eine elastische Verformung und Rückstellung, sodass der Einstellring 9 auf einfache Weise in verschiedene Positionen gedreht werden kann. Der Einstellring 9 kann zumindest eine Anzeigevorrichtung 28 zur Identifikation des Fingers enthalten.

Der Einstellring 9 lässt sich in der Ringführung 12 drehen, um das gewünschte Anzeigeelement 28 unterhalb der Markierung 27 zu setzen. Die Position der Markierung 27 des Messkopfs 5 und das Anzeigeelement 28 auf dem Einstellring 9 befinden sich in der gewünschten Position, sodass die gewünschte Fingerbezeichnung eingestellt wird und entsprechend angezeigt wird. Mit der eingestellten Fingerbezeichnung kann die Fingerposition der rechten oder linken Hand später dem fotografisch ermittelten Abbild der Papillarleisten unmittelbar und eindeutig zugeordnet werden.

Fig. 4g zeigt ein Detail eines Einstellrings 9 nach dem in Fig. 4a bis Fig. 4f gezeigten dritten Ausführungsbeispiel in einer Ansicht von oben. Fig. 4g zeigt eine Ansicht des Einstellrings 9 gemäss Fig. 4g und dessen Halterung 30 in einer Explosionsdarstellung. Nach dem vorliegenden Ausführungsbeispiel ist der Einstellring 9 im zusammengebauten Zustand in der Halterung 30 aufgenommen. Die Halterung 30 ist auf einem an den Mantel 4 anschliessenden Deckenelement 29 befestigt. Das Deckenelement 29 ist insbesondere in Fig. 4f deutlich zu sehen. Die Halterung 30 weist eine zentrale Ausnehmung 31 auf, die nach dem vorliegenden Ausführungsbeispiel auf einen entsprechenden Vorsprung 32 des Deckenelements 29 aufgesteckt werden kann.

Die Ausnehmung 31 und der Vorsprung 32 weisen korrespondierende Querschnitte auf. Nach dem vorliegenden Ausführungsbeispiel ist die Ausnehmung als Vierkantöffnung und der Vorsprung als Vierkant ausgebildet. Nach einem nicht dargestellten Ausführungsbeispiel ist die Ausnehmung als sechseckige Öffnung und der Vorsprung als Sechskant ausgebildet. Nach einem weiteren nicht dargestellten Ausführungsbeispiel kann die Ausnehmung die Form einer Ellipse aufweisen und der Vorsprung als ein Stiftelement mit entsprechendem ovalem Querschnitt ausgebildet sein. Somit weisen die Ausnehmung 31 und der Vorsprung 32 korrespondierende nicht rotationssymmetrische Querschnitte auf, sodass eine Drehbewegung der Halterung 30 relativ zum Deckenelement 29 ausgeschlossen ist.

Die Ausnehmung 31 ist in einem Grundkörper 33 der Halterung 30 angeordnet. Der Grundkörper 33 enthält eine Mehrzahl von Armelementen 34, die vom Grundkörper 33 abstehend angeordnet sind. Im vorliegenden Ausführungsbeispiel sind exemplarisch fünf Armelemente 34 gezeigt, es können aber mindestens drei Armelemente und auch mehr als fünf Armelemente 34 vorgesehen werden. Jedes der Armelemente 34 weist einen ersten Endbereich 35 und einen zweiten Endbereich 36 auf. Der erste Endbereich 35 ist mit dem Grundkörper 33 fest verbunden. Der zweite Endbereich 36 enthält nach dem vorliegenden Ausführungsbeispiel eine nasenartige Verdickung 37. Die nasenartige Verdickung 37 befindet sich auf der Aussenseite des zweiten Endbereichs 36. Die nasenartige Verdickung 37 ist nach dem vorliegenden Ausführungsbeispiel zum Eingriff in entsprechende nasenartige Aussparungen 38 des Einstellrings 9 bestimmt. Die Halterung 30 bildet somit mit dem Einstellring nach dem vorliegenden Ausführungsbeispiel eine Ratsche. Mit anderen Worten rasten die nasenartigen Verdickungen 37 in die entsprechenden nasenartigen Aussparungen 38 des Einstellrings 9 ein, sodass der Einstellring 9 in der gewünschten Position festgestellt werden kann, was in Fig. 4g gezeigt ist.

An der Aussenseite des Einstellrings 9 ist zumindest ein Anzeigeelement 28 angeordnet. Insbesondere kann eine Mehrzahl von Anzeigeelementen 28 vorgesehen werden. Beispielsweise können die Anzeigeelemente 28 analog zu den vorhergehenden Ausführungsbeispielen die international standardisierten Fingerbezeichnungen enthalten. Daher sind nach einem bevorzugten Ausführungsbeispiel zehn Anzeigeelemente 28 vorgesehen. Die Anzahl der nasenartigen Aussparungen 38 kann insbesondere der Anzahl der Anzeigeelemente 28 entsprechen.

Die nasenartige Aussparung 38 kann insbesondere einen ersten Schenkel 41 und einen zweiten Schenkel 42 enthalten. Insbesondere kann sich der Winkel, den der erste Schenkel 41 mit einer Grundlinie 40 einschliesst, vom Winkel, den der zweite Schenkel 42 mit der Grundlinie 40 einschliesst, unterscheiden. Als Grundlinie 40 wird die Gerade bezeichnet, welche den Schnittpunkt 43 des ersten Schenkels 41 mit der Innenkante des Einstellrings 9 und den Schnittpunkt 44 des zweiten Schenkels 42 mit der Innenkante des Einstellrings 9 verbindet. Die Grundline 40 ist für eine der nasenartigen Aussparungen 38 strichliert in Fig. 4g eingetragen. Nach dem vorliegenden Ausführungsbeispiel ist der Winkel, den der erste Schenkel 41 mit der Grundlinie 40 einschliesst, kleiner als der Winkel, den der zweite Schenkel 42 mit der Grundlinie 40 einschliesst. Der erste Schenkel 41 schliesst somit einen weniger steilen Winkel mit der Grundlinie 40 ein als der zweite Schenkel 42. Dieses Ausführungsbeispiel ermöglicht, eine Vorzugsdrehrichtung zu definieren. In der Vorzugsdrehrichtung können die nasenartigen Verdickungen 37 einfach aus den nasenartigen Aussparungen 38 entfernt werden, in der Gegenrichtung würde die Drehung durch die steileren zweiten Schenkel 42 blockiert werden.

Für eine Fachperson ist offensichtlich, dass viele weitere Varianten zusätzlich zu den beschriebenen Ausführungsbeispielen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C bis N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Eine Vorrichtung (10) zur fotografischen Erfassung der Papillarleisten einer Fingerkuppe einer verstorbenen Person umfassend einen Messkopf (5), wobei der Messkopf (5) zur Aufnahme einer Fingerenglieds ausgebildet ist, wobei der Messkopf (5) einen Mantel (4) enthält, wobei der Mantel (4) einen ersten Endbereich (1) und einen zweiten Endbereich (2) und eine Mittenachse (3) enthält, wobei der erste Endbereich (1) des Messkopfs (5) als zumindest teilweise geschlossene Oberfläche ausgebildet ist, wobei der zweite Endbereich (2) des Messkopfs (5) einen Messring (6) umfasst, wobei ein Messfenster (7) im Mantel (4) des Messkopfs (5) zwischen dem ersten Endbereich (1) und dem zweiten Endbereich (2) angeordnet ist, **dadurch gekennzeichnet, dass** ein Griffelement (8) sich vom zweiten Endbereich (2) des Messkopfs (5) im Wesentlichen in Richtung der Mittenachse (3) erstreckt.

2. Vorrichtung nach Anspruch 1, wobei das Griffelement (8) als ein rinnenförmiges Auflageelement ausgebildet ist.

3. Vorrichtung nach Anspruch 2, wobei das Griffelement (8) eine Länge LG aufweist, wobei die Länge LG grösser als eine Länge LM des Messkopfs (5) ist.

4. Vorrichtung nach einem der Ansprüche 2 oder 3, wobei das Griffelement (8) einen Griffelementabschnitt (13) mit einer Einschnürung (15) enthält.

5. Vorrichtung nach einem der Ansprüche 2 bis 4, wobei das Griffelement (8) ein Markierungselement (14) enthält.

6. Vorrichtung nach einem der vorhergehenden Ansprüche, wobei der Messkopf (5) als ein rotationssymmetrischer Körper ausgebildet ist.

7. Vorrichtung nach einem der vorhergehenden Ansprüche, wobei sich das Messfenster (7) über einen Winkelbereich eines Umfangs des Mantels (4) erstreckt, wobei der Winkelbereich im Bereich von 170 Grad bis einschliesslich 190 Grad liegt.

8. Vorrichtung nach einem der vorhergehenden Ansprüche, wobei ein Einstellring (9) zwischen dem Messkopf (5) und dem Mantel (4) angeordnet ist.

9. Vorrichtung nach Anspruch 8, wobei der Mantel (4) ein Deckenelement (29) enthält, wobei ein Vorsprung (32) derart auf dem Deckenelement (29) angeordnet ist, dass der Vorsprung dem Messkopf (5) gegenüberliegt.

10. Vorrichtung nach einem der Ansprüche 8 oder 9, wobei der Einstellring (9) mit einer Halterung (30) derart zusammenwirkt, sodass durch den Einstellring (9) und die Halterung (30) im zusammengebauten Zustand eine Ratsche ausgebildet ist.

11. Vorrichtung nach Anspruch 10, wobei die Halterung (30) eine Ausnehmung (31) aufweist, die zur Aufnahme im Vorsprung (32) ausgebildet ist.

12. Vorrichtung nach Anspruch 11, wobei der Vorsprung (32) und die Ausnehmung (31) korrespondierende, insbesondere nicht rotationssymmetrische Querschnitte aufweisen.

13. Vorrichtung nach einem der Ansprüche 10 bis 12, wobei die Halterung (30) eine Mehrzahl von Armelementen (34) enthält, die sich von einem ersten Endbereich (35), der mit einem Grundkörper (33) der Halterung (30) verbunden ist, zu einem zweiten Endbereich (36) erstrecken, wobei im zweiten Endbereich (36) jedes Armelements (34) je eine nasenartige Verdickung (37) angeordnet ist, die zum Eingriff in je eine korrespondierende, am Einstellring (9) angeordnete nasenartige Aussparung (38), ausgebildet ist.

14. Vorrichtung nach einem der Ansprüche 8 bis 13, wobei der Einstellring (9) auf der Innenseite eine Mehrzahl von Ausnehmungen (25) für je ein Magnetelement enthält.

15. Vorrichtung nach einem der Ansprüche 8 bis 14, wobei der Einstellring (9) zumindest ein Anzeigeelement (28) zur Identifikation des Fingers enthält.
